# EUROPEAN PATENT APPLICATION

(11) **EP 1 913 936 A1**
(43) Date of publication of application: **23.04.2008**
(21) Application number: 06757780.9
(22) Date of filing: 06.06.2006
(51) Int. Cl.: A61K 9/20, A61K 31/541

(54) **SUBLINGUAL SOLID PHARMACEUTICAL FORMULATIONS CONTAINING MELOXICAM**

(30) Priority: 12.08.2005 MX PA05008575
(71) Applicant: ESPINOZA ABDALA, Leopoldo de Jesús, C.P. 45110 Zapopan, Jalisco, Mexico (MX)
(72) Inventor: GARCÍA ARMENTA, María Elena, C.P. 45020 Guadalajara, Jalisco, México (MX); GARCÍA ARMENTA, Patricia, C.P. 45110 Zapopan, Jalisco, México (MX); ÁLVAREZ OCHOA, Víctor Guillermo, C.P. 45222 Zapopan, Jallisco, México (MX); SANTOS MURILLO, Josefina, C.P. 44600 Zapopan, Jalisco, México (MX); GARCÍA ALCALÁ, Martín Omar, C.P. 44410 Guadalajara, Jalisco, México (MX); ESPINOSA ABDALA, Leopoldo de Jesus, Colinas de San Javier C.P. 45110 Zapopan, Jalisco (MX)
(74) Representative: Carvajal y Urquijo, Isabel
(86) International application number: PCT/MX2006/000047
(87) International publication number: WO 2007/021168

(57) **Abstract**

The invention relates to the pharmaceutical industry in general and to the pharmaceutical industry involved in the production of various different sublingual solid pharmaceutical formulations containing meloxicam. The inventive pharmaceutical formulations are **characterized in that** they comprise: (a) meloxicam, (b) one or more anti-adherents, (c) one or more disintegrating agents, (d) one or more binding agents, (e) one or more lubricants, (f) one or more diluent agents, (g) one or more solvents, (h) one or more solubilising agents, (i) one or more sweetening agents, (j) one or more flavouring agents and/or essences, (k) one or more viscosity agents, (1) one or more antimicrobial agents, (m) one or more surfactants, (n) one or more antioxidants, (o) one or more emulsifying agents, and (p) any other additive that helps the formulation.

## Description

### FIELD OF THE INVENTION

The present invention is related generally to the pharmaceutical industry, and particularly to the pharmaceutical industry that produces various sublingual solid pharmaceutical formulations containing Meloxicam.

### BACKGROUND OF THE INVENTION

Solid pharmaceutical forms are preparations containing the active principle(s) and additives, generally disc-shaped, scored or non-scored, molded and of variable size obtained by compressing powders or granules into tablets, capsules, patches, pessaries, beads, suppositories, troches or lozenges.

Lumbalgia represents one of the reasons of more frequent consultation in Primary Attention and one of the main causes of labor absenteeism.

Osteoarthritis is the eighth cause of disability worldwide, according to studies realized by WHO. It is the most frequent of all joint diseases and its prevalence increases with age. Typical arthrosis joint disturbances begin in the second decade of life, affecting about 90% of people above 40 years old.

The illness is distributed universally, although there are geographical differences, due in part to genetic and environmental factors and to the different usage of the joints.

Rheumatoid arthritis is a disorder having the potential to severely affect survival, functionality and quality of life of the individual suffering from it, as well as the ability to keep a satisfactory employment. Mortality due to direct causes or by complications derived from rheumatoid arthritis is still almost twice than observed in the control population, and this trend has kept unchanged for the last four decades. It affects from 0.5% to 1.0% of world population.

Therefore, the present invention provides new formulations comprising: (a) Meloxicam, (b) one or more anti-adherent agents, (c) one or more disintegrating agents, (d) one or more binding agents, (e) one or more lubricants, (f) one or more diluting agents, (g) one or more solvents, (h) one or more solubilizing agents, (i) one or more sweeteners, (j) one or more flavoring and/or perfuming agents, (k) one or more viscosity agents, (1) one or more antimicrobial agents, (m) one or more surfactants, (n) one or more antioxidants, (o) one or more emulsifiers, and (p) any other additive required for the formulation.

Meloxicam is 4-hydroxy-2-methyl-N-(5-methyl-2-thiazolyl)-2H-1,2-benzothiazine-3-carboxamide-1,1-dioxide and can be represented by formula (I):

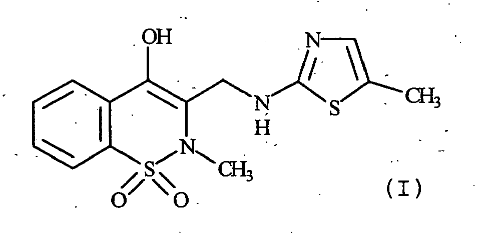

Compound of formula (I) is known by its antiinflammatory, analgesic and antipyretic activity,_ which acts by inhibiting the synthesis of prostaglandins with' greater potency at the site of inflammation than on kidney and gastric mucosa. Currently is indicated, in the treatment of rheumatoid arthritis, osteoarthritis, periarthritis in scapulohumeral and coxofemoral joints, muscular strains, and pain and inflammation in soft tissues and airways having inflammatory processes.

### SUMMARY OF THE INVENTION

Briefly, the present invention can be reflected into a medicament which drug concentration in the formulation is in an amount from 0.0001% to 95.0% w/w, preferably from 0.5 to 50.0% w/w for Meloxicam. This drug present in said formulation could be found as its anhydrous or .hydrated base such as free meloxicam base or as a physiologically acceptable salt.

This invention relates to sublingual solid formulations containing Meloxicam.

The present invention provides new formulations comprising: (a) Meloxicam, (b) one or more anti-adherent agents, (c) one or more disintegrating agents, (d) one or more binding agents, (e) one or more lubricants, (f) one or more diluting agents, (g) one or more solvents, (h) one or more solubilizing agents, (i) one or more sweeteners, (j) one or more flavoring and/or perfuming agents, (k) one or more viscosity agents, (1) one or more antimicrobial agents, (m) one or more surfactants, (n) one or more antioxidants, (o) one or more emulsifiers, and (p) any other additive required for the formulation.

These additives or excipients consisting in, for example, diluting agents such as lactose, mannitol, dextrose, sucrose, calcium phosphate, microcrystalline cellulose, calcium sulfate, kaolin, compressible sugar, corn starch, among others. The diluting agent could be present in an amount from 5% to 99%.

The disintegrating agents are selected from corn starch, alginic acid, celluloses and their derivatives, povidone, sodium crosscarmelose, starch sodium glycolate, among others. The disintegrating agent could be present in an amount from 0.0001% to 25.0%.

Binding agents such as polyvynilpirrolidone, tragacanth, acacia, starch, methyl cellulose, among others. The binding agent could be present in an amount from 0.01% to 10.0%.

The polar and non-polar dissolving agent can be: water, ethyl alcohol, acetone, isopropyl myristate, polyoxypropylenes, propylene glycol, polyethylene glycol, glycerol, 70% sorbitol, polyethylene glycols, mineral oil, petrolatum, lanoline, vegetable waxes, animal waxes, vegetable oils such as olive oil, cottonseed oil, corn oil, among others, or a combination thereof, with ethyl alcohol, 70% sorbitol solution, vegetable oils and polar solvents such as water being the more preferred to be used. The final formulation can contain from 1% to 95% w/v of the dissolvent.

Lubricating agents are selected from stearic acid, stearate and talc, among others. The lubricating agent can be present in a ratio from 0.0001% to 10.0%.

Anti-adhering agents such as, colloidal silicon dioxide, calcium sulfate, calcium chloride, talc, corn starch, among others. The anti-adherent can be present in a ratio from 0.0001% to 10.0%.

Solubilizing agents such as povidone, cyclodextrins, n-methylglucamine, lecithin, monoethanolamine, glycine, benzyl benzoate, poloxamers, polyoxyethylene alquil ethers, among others. The solubilizing agent can be present in a ratio from 0.0001% to 50.0 %.

One or more surfactants such as sodium laurate, sodium oleate, sodium laurylsulfate, sodium cholate, sodium deoxycholate, sodium diamylsulfosuccinate, sodium dioctylsulfosuccinate, poloxamers, lecithin, tetradecyltrimethylammonium bromide, hexadecylpyridinium chloride, polysorbate 20, polysorbate 60, among others. The surfactant can be present in a ratio from 0.0001% to 30%.

Emulsifying agents such as Arabic gum, tragacanth gum, alginates, chondrus, pectin, aluminum silicate, bentonite, aluminum magnesium silicate, sodium dodecylsulfate, benzalconium chloride, polyoxyethylene sorbitan monostereate, polyethylene glycol 400 monostearate, ethylene glycol distearate, sorbitan tristearate, sorbitan monopalmitate, diethylene glycol monostearate, sodium oleate, potassium oleate, sodium lauryl sulfate, among others. The emulsifying agent can be present from 0.0001% to 10%.

Sweetening agents such as aspartame, acesulfame k, dextrose, fructose, glucose, mannitol, sorbitol, sugar, sucrose, among others. The sweetening agent can be present in a ratio from 0.0001% to 60.0%.

Flavoring agents, such as menthol, vanillin, cinnamon, sorbitol, citric acid, cherry flavor, orange flavor, pineapple flavor, peach flavor, grape flavor, strawberry flavor, among others. The flavoring agent can be present in a ratio from 0.0001% to 5.0%.

Antioxidant agents such as disodium edetate (EDTA), ascorbic acid, butylhydroxytoluene (BHT), tocopherols, sodium bisulfite or sodium metabisulfite, gallic acid, propylgallate, ascorbyl palmitate, among others. The antioxidant agent can be present in a ratio from 0.0001% to 20.0%.

Antimicrobial agents such as sorbic acid; potassium sorbate; potassium benzoate; chlorobutanol; methyl p-hydroxybenzoate; propyl p-hydroxybenzoate; thimerosal, among others. The antimicrobial agent can be present in a ratio from 0.0001% to 5.0% w/v.

Viscosifying agents such as acacia, agar, alginic acid, povidone, hydroxypropylmethyl cellulose, sodium carboxymethyl cellulose, calcium carboxymethylcellulose, pectin, gelatin, guar gum, xanthan gum, carbomer, bentonite, hydroxypropylcellulose, among others, preferably sodium carboxymethyl cellulose. The final formulation can contain from 0.0001% to 10.0% of the viscosifying agent.

The formulation can also contain other components such as a flocculating agent like sodium chloride or potassium chloride; a pH buffering system such as phosphates, phosphoric acid, citrates, citric acid, carbonates, bicarbonates, acetates, lactates, among others; they can be present as its anhydrous or hydrated base or as a physiologically acceptable salt. The flocculant agent can be found in a ratio from 0.001% to 5.0% w/v and the pH buffering system can be present in a proportion from 0.01% to 10% w/w.

The present invention also provides a method for the treatment of processes exhibiting inflammation and pain such as lumbar pain, cervialgias, brachialgias, radiculitis, peripheral neuropathies of diverse ethiopathogenesis: facial neuralgia, trigeminal neuralgia, intercostals neuralgia, herpetic neuralgia, alcoholic neuropathy, diabetic neuropathy, carpian tunnel syndrome, fibromyalgia, spondylitis, rheumatoid arthritis, osteoarthritis, periarthritis in scapulohumeral and coxofemoral joints, muscular strains, and pain and inflammation in soft tissues and airways, originated by different ethyology, by administering suitable doses of the instant formulations.

The formulations can be contained in containers having suitable capacity varying from 5 ml to 150 ml elaborated from high and/or low density polyethylene, polyethylene terephthalate, polyvinyl chloride, polypropylene, polystyrene, Type I, II, III and IV glass, among others, colored or non colored. The cap may be of the following types: tamperproof, threaded, cap-to-cap, child proof, elaborated from high and/or low density polyethylene, polyethylene terephthalate, polyvinyl chloride, polypropylene, polystyrene, colored or non colored. In a blisterpack made of PVC (polyvinyl chloride) film with a thickness from 200 µ to 250 µ, which optionally can have a coating of PVDC (polyvinylidene chloride) with a weight from 25 g/m² to 120 g/m², and bonded with aluminum foil or in cellopolyal film.

The elaboration of the different pharmaceutical forms is carried out mixing the active ingredients with the corresponding additives, in the proper concentrations.

The administration of 15 mg meloxicam every 24 hours give rise to peak concentrations of 1.6 µg/ml in plasma. Concentrations at equilibrium can be reached in 3 to 5 days. 99% of meloxicam binds to plasma proteins. The drug has a good distribution in the body, but particularly it achieves high penetration in synovial fluid, reaching levels that are equivalent to the half of concentrations in plasma. Meloxicam is metabolized mainly through oxidation of the methyl group in the thiazolyl molecule. About 50% of the dose is eliminated through urine and the remaining is excreted in feces. The clearance half-life is 20 hours.

For treatment of pain, the use of 7.5 mg or 15 mg every 12 or 24 hours is recommended, as judged appropriate by the physician.

Therefore, the present invention provides a sublingual solid pharmaceutical formulation, which provides from 0.25 mg to 25.0 mg of Meloxicam in suitable doses.

### EXAMPLES

The following examples with illustrative but nonlimiting character will assist to illustrate the present invention:

### EXAMPLE 1

| | | |
|---|---|---|
| 1) | Meloxicam | 40.00% w/w |
| 2) | anhydrous lactose | 35.00% w/w |
| 3) | Sucrose | 3.00% w/w |
| 4) | 96° Ethyl alcohol | 19.20% w/v |
| 8) | Purified water | 12.80% w/v |

The preparation of tablets is carried out as follows: Meloxicam is mixed with anhydrous lactose and sucrose, in a separated vessel alcohol and water are mixed; this solution is gradually added to the first mixture. Finally, molding is conducted.

### EXAMPLE 2

| | | |
|---|---|---|
| 1) | Meloxicam | 30.00% w/w |
| 2) | anhydrous lactose | 45.00% w/w |
| 3) | Sucrose | 5.00% w/w |
| 4) | 96° Ethyl alcohol | 12.00% w/v |
| 8) | Purified water | 8.00% w/v |

The preparation of tablets is carried out as follows: Meloxicam is mixed with anhydrous lactose, in a separated vessel alcohol and water are mixed; in this solution sucrose is dissolved and then this solution is gradually added to the first mixture. Finally, the molding is carried out.

### EXAMPLE 3

| | | |
|---|---|---|
| 1) | Meloxicam | 30.00% w/w |
| 2) | anhydrous lactose | 64.00% w/w |
| 3) | Polyethylene glycol 4000 | 0.70% w/w |
| 4) | 96° Ethyl alcohol | 3.18% w/v |
| 8) | Purified water | 2.12% w/v |

The preparation of tablets is carried out as follows: Meloxicam is mixed with anhydrous lactose, in a separated vessel alcohol and water are mixed; in this solution Polyethylene glycol 4000 is dissolved and then this solution is gradually added to the first mixture. Finally, the molding is carried out.

These formulations are indicated for treatment of' processes exhibiting inflammation and pain such as lumbar pain, cervialgias, brachialgias, radiculitis, peripheral neuropathies of diverse ethiopathogenesis: facial neuralgia, trigeminal neuralgia, intercostals neuralgia, herpetic neuralgia, alcoholic neuropathy, diabetic neuropathy, carpian tunnel syndrome, fibromyalgia, spondylitis, rheumatoid arthritis, osteoarthritis, periarthritis in scapulohumeral and coxofemoral joints, muscular strains, and pain and inflammation in soft tissues and airways, originated by different ethyology, by administering suitable doses of the instant formulations.

The invention has been described sufficiently as to allow that a person of ordinary skill in the art could realize and obtain the results mentioned in the present invention. However, any person of ordinary skill in the art to which the present invention pertains could be able to make modifications not disclosed in the present application, nonetheless, if for the application of these modifications in a given structure or in the manufacturing process thereof, the subject matter claimed in the following claims is required, said structures should be embraced within the scope of the invention.

## Claims

1. Sublingual solid pharmaceutical formulations containing Meloxicam, wherein said formulations comprise: (a) Meloxicam, (b) one or more anti-adherent agents, (c) one or more disintegrating agents, (d) one or more binding agents, (e) one or more lubricants, (f) one or more diluting agents, (g) one or more solvents, (h) one or more solubilizing agents, (i) one or more sweeteners, (j) one or more flavoring and/or perfuming agents, (k) one or more viscosity agents, (1) one or more antimicrobial agents, (m) one or more surfactants, (n) one or more antioxidants, (o) one or more emulsifiers, and (p) any other additive required for the formulation.

2. Sublingual solid pharmaceutical formulations containing Meloxicam as claimed in the preceding claim, wherein drug concentration in the formulation is in an amount from 0.0001% to 95.0% w/w, preferably from 0.5 to 50.0% w/w for Meloxicam.

3. Sublingual solid pharmaceutical formulations containing Meloxicam as claimed in the preceding claim, wherein the drugs combined therein could be found as its base or as a physiologically acceptable salt, with free meloxicam base being preferred.

4. Sublingual solid pharmaceutical formulations containing Meloxicam as claimed in any one of the preceding claims, wherein said solid pharmaceutical forms contain diluting agent selected from lactose, mannitol, calcium phosphate, microcrystalline cellulose, calcium sulfate, compressible sugar, corn starch, kaolin, or a combination thereof; a disintegrating agent selected from corn starch, alginic acid, celluloses and their derivatives, povidone, sodium crosscarmelose, starch sodium glycolate, or a combination thereof; binding agents selected from polyvidone, tragacanth, acacia, starch, methyl cellulose, or a combination thereof; polar and non-polar dissolving agents selected from water, ethyl alcohol, acetone, isopropyl myristate, polyoxypropylenes, propylene glycol, polyethylene glycol, glycerol, 70% sorbitol, polyethylene glycols, mineral oil, petrolatum, lanoline, vegetable waxes, animal waxes, vegetable oils such as olive oil, cottonseed oil, corn oil, or a mixture thereof; lubricating agents selected from stearic acid, stearate and talc, or a mixture thereof; anti-adhering agents selected from colloidal silicon dioxide, calcium sulfate, calcium chloride, talc, corn starch, among others.

5. Sublingual solid pharmaceutical formulations containing Meloxicam as claimed in any one of the preceding claims, wherein said solid pharmaceutical forms can be contained in containers of suitable capacity, elaborated from a material selected from high and/or low density polyethylene, polyethylene terephthalate, polyvinyl chloride, polypropylene, polystyrene, Type I, II, III and IV glass, among others, colored or non colored; with a cap selected from a tamperproof cap, threaded cap, cap-to-cap, child proof cap, elaborated from high and/or low density polyethylene, polyethylene terephthalate, polyvinyl chloride, polypropylene, polystyrene, colored or non colored, or in a container selected from a blisterpack made of PVC (polyvinyl chloride) film with a thickness from 200 µ to 250 µ, which optionally can have a coating of PVDC (polyvinylidene chloride) with a weight from 25 g/m² to 120 g/m², and bonded with aluminum foil or in cellopolyal film.

6. Sublingual solid pharmaceutical formulations containing meloxicam as claimed in any one of the preceding claims, wherein said solid pharmaceutical contains polar and non-polar dissolving agents selected from water, ethyl alcohol, acetone, isopropyl myristate, polyoxypropylenes, propylene glycol, polyethylene glycol, glycerol, 70% sorbitol, polyethylene glycols, mineral oil, petrolatum, lanoline, vegetable waxes, animal waxes, vegetable oils such as olive oil, cottonseed oil, corn oil, or a mixture thereof; solubilizing agents selected from povidone, cyclodextrins, n-methylglucamine, lecithin, monoethanolamine, glycine, benzyl benzoate, poloxamers, polyoxyethylene alquil ethers and a mixture thereof; surfactants such as sodium laurate, sodium oleate, sodium laurylsulfate, sodium cholate, sodium deoxycholate, sodium diamylsulfosuccinate, sodium dioctylsulfosuccinate, poloxamers, lecithin, tetradecyltrimethylammonium bromide, hexadecylpyridinium chloride, polysorbate 20, polysorbate 60, or a mixture thereof; emulsifying agents selected from Arabic gum, tragacanth gum, alginates, chondrus, pectin, aluminum silicate, bentonite, aluminum magnesium silicate, sodium dodecylsulfate, benzalconium chloride, polyoxyethylene sorbitan monostereate, polyethylene glycol 400 monostearate, ethylene glycol distearate, sorbitan tristearate, sorbitan monopalmitate, diethylene glycol monostearate, sodium oleate, potassium oleate, sodium lauryl sulfate, and a mixture thereof; sweetening agents selected from aspartame, acesulfame k, dextrose, fructose, glucose, mannitol, sorbitol, sugar, sucrose; flavoring agents, such as menthol, vanillin, cinnamon, sorbitol, citric acid, cherry flavor, orange flavor, pineapple flavor, peach flavor, grape flavor, strawberry flavor; antioxidant agents such as disodium edetate (EDTA), ascorbic acid, butylhydroxytoluene (BHT), tocopherols, sodium bisulfite or sodium metabisulfite, gallic acid, propylgallate, ascorbyl palmitate; antimicrobial agents such as sorbic acid; potassium sorbate; potassium benzoate; chlorobutanol; methyl p-hydroxybenzoate; propyl p-hydroxybenzoate; thimerosal; viscosifying agents selected from acacia, agar, alginic acid, povidone, hydroxypropylmethy cellulose, sodium carboxymethyl cellulose, calcium carboxymethylcellulose, pectin, gelatin, guar gum, xanthan gum, carbomer, bentonite, hydroxypropylcellulose, among others.

7. Sublingual solid pharmaceutical formulations containing Meloxicam as claimed in the preceding claim, wherein said solvents are ethyl alcohol, 70% sorbitol solution, vegetable oils and polar solvents such as water.

8. Sublingual solid pharmaceutical formulations containing meloxicam as claimed in any one of the preceding claims, wherein said solid pharmaceutical forms can also contain other components such as a flocculating agent like sodium chloride or potassium chloride, dextrose, mannitol; a pH buffering system such as phosphates, citrates, carbonates, bicarbonates, acetates, lactates; alcalinizing or acidifying agents such as sodium hydroxide, sodium bicarbonate, monoethanolamine, triethanolamine, hydrochloric acid, citric acid, acetic acid and binding agents such as polyvidone, tragacanth, acacia, starch, and methyl cellulose.

9. Sublingual solid pharmaceutical formulations containing Meloxicam as claimed in any one of the preceding claims, wherein said formulations provided from 0.25 mg to 25 mg of Meloxicam in suitable doses.
